# EUROPEAN PATENT APPLICATION

(11) **EP 4 643 808 A1**
(43) Date of publication of application: **05.11.2025**
(21) Application number: 23910987.9
(22) Date of filing: 29.12.2023
(51) Int. Cl.: A61C 7/00, G06T 7/00, G06T 7/68, G06T 7/73, G06T 17/00

(54) **METHOD FOR GENERATING FACIAL MIDLINE, ORTHODONTIC APPLIANCE, AND MANUFACTURING METHOD THEREFOR**

(30) Priority: 30.12.2022 CN 202211740579
(71) Applicant: Shanghai Ea Medical Instruments Company Limited, Shanghai 200433 (CN)
(72) Inventor: WANG, Jing, Shanghai 200433 (CN); WANG, Mingzheng, Shanghai 200433 (CN); FENG, Yang, Shanghai 200433 (CN)
(74) Representative: Ipsilon
(86) International application number: PCT/CN2023/143506
(87) International publication number: WO 2024/141074

(57) **Abstract**

A method for generating a facial midline, an orthodontic appliance, and a manufacturing method therefor. The method for generating a facial midline comprises obtaining a facial image of a patient; performing recognition on the facial image to determine image deviation information of a facial midline and a dental midline of the patient in the facial image; and utilizing the image deviation information and physical parameter information of a dental model of the patient to generate a facial midline on the dental model.

## Description

### METHOD THEREFOR

This application claims priority to Chinese Patent Application No. 2022117405796, filed on December 30, 2022, the content of which is hereby incorporated by reference in its entirety.

### Technical Field

The present application relates to the technical field of dental orthodontic treatment, and in particular to a method for generating a facial midline, an orthodontic treatment appliance, and a manufacturing method therefor.

### Background

Based on the concept of oral aesthetics, more and more people have a higher pursuit of their own tooth shape, and orthodontic treatment (also known as orthodontics) has emerged. In a process of orthodontic treatment, it is necessary to adjust a position of each tooth according to certain rules. For example, a position of a facial midline of a face of a person to be treated is used as a reference, and whether a tooth is adjusted to the left or right relative to the facial midline is determined according to a relative relationship between a position of a dental midline and the facial midline.

In an existing orthodontic treatment process, an orthodontic designer needs to observe a two-dimensional tooth-exposed photo of a person to be treated, roughly estimate the position of the facial midline in a dentition in combination with personal experience, and then manually estimate the position of the facial midline in a three-dimensional dental model including a crown and a gum of the person to be treated. The method not only has a complicated process, but also has a low accuracy of the facial midline determined, and has a certain randomness and error.

### Summary

The technical problem to be solved in the present application is to overcome the need for manual estimation of the position of the facial midline in the three-dimensional dental model during the orthodontic treatment process in related art. The process of determining the facial midline is complicated, and the accuracy of the midline determined is low, with a certain randomness and error. The present application provides a method for generating a facial midline, an orthodontic treatment appliance, and a manufacturing method.

In a first aspect, embodiments of the present application provide a method for generating a facial midline, the method includes:
obtaining a facial image of a patient;
recognizing the facial image and determining image deviation information between a facial midline and a dental midline of the patient in the facial image; and
generating a facial midline on a dental model of the patient according to the image deviation information and physical parameter information of the dental model.

In a possible implementation, physical deviation information between the facial midline and the dental midline of the patient is determined according to the image deviation information and physical size information of a target tooth; the facial midline is generated on the dental model according to physical deviation information between the facial midline and the dental midline of the patient.

In a possible implementation, the target tooth is at least a portion of at least one tooth associated with the facial midline and the dental midline.

In a possible implementation, the facial image at least partially includes a partial image of at least one tooth of the patient; the determining the image deviation information between the facial midline and the dental midline of the patient in the facial image, includes: recognizing facial features of the facial image and obtaining position information of the facial midline of the patient in the facial image; recognizing the tooth in the facial image and determining position information of the dental midline; determining the image deviation information according to the position information of the facial midline and the position information of the dental midline, where the image deviation information includes: image pixel information of the facial midline and the dental midline.

In a possible implementation, a tooth in the facial image is recognized and boundary information of the target tooth is determined; where the boundary information includes: pixel information of a dental image; a mapping relationship is determined according to the boundary information of the target tooth and physical width information of the target tooth, where the mapping relationship represents a mapping relationship between an image pixel and a physical space of the dental model; the mapping relationship is used for generating the facial midline on the dental model.

In a possible implementation, the physical width information corresponding to the target tooth is obtained in the dental model; a pixel value occupied by the target tooth in the facial image in a first direction is obtained according to the boundary information of the target tooth; where the first direction is associated with the physical width information; the mapping relationship is determined according to the physical width information and the pixel value.

In a possible implementation, the physical deviation information between the facial midline and the dental midline of the patient is determined according to the mapping relationship, dental midline information of the dental model, and the image deviation information;
or, the facial midline on the dental model is determined according to the mapping relationship, the image deviation information, and dental midline information of the dental model.

In a possible implementation, the facial midline on the dental model is used for generating or adjusting an orthodontic treatment plan for the patient;
and/or, the physical deviation information between the facial midline and the dental midline of the patient is used for generating or adjusting an orthodontic treatment plan for the patient.

In a possible implementation, the facial midline generated is displayed on the dental model.

In a second aspect, the present application provides a method for generating an orthodontic treatment plan, a facial midline generated on a dental model is obtained, where the facial midline is generated based on the method for generating the facial midline in the first aspect; and an orthodontic treatment plan for the patient is generated according to the facial midline and the dental model of the patient.

In a third aspect, the present application provides a method for generating an orthodontic treatment plan, the method includes:
obtaining a facial image of a patient;
recognizing the facial image and determining image deviation information between a facial midline and a dental midline of the patient in the facial image;
determining physical deviation information between the facial midline and the dental midline of the patient according to the image deviation information and physical parameter information of a tooth of the patient; and
generating an orthodontic treatment plan for the patient based on the physical deviation information and a dental model of the patient.

In a possible implementation, the physical deviation information between the facial midline and the dental midline of the patient is determined according to the image deviation information and physical size information of a target tooth, where the target tooth is at least a portion of at least one tooth associated with the facial midline and the dental midline.

In a possible implementation, the facial image at least partially includes a partial image of at least one tooth of the patient; the determining the image deviation information between the facial midline and the dental midline of the patient in the facial image includes:
recognizing facial features of the facial image and obtaining position information of the facial midline of the patient in the facial image;
recognizing the tooth in the facial image and determining position information of the dental midline;
determining the image deviation information according to the position information of the facial midline and the position information of the dental midline, where the image deviation information includes: image pixel information of the facial midline and the dental midline.

In a possible implementation, a tooth in the facial image is recognized and boundary information of the target tooth is determined; where the boundary information includes: pixel information of a dental image;
a mapping relationship is determined according to the boundary information of the target tooth and physical width information of the target tooth, where the mapping relationship represents a mapping relationship between an image pixel and a physical space of the dental model; the mapping relationship is used for generating the facial midline on the dental model.

In a possible implementation, the physical width information corresponding to the target tooth is obtained in the dental model;
a pixel value occupied by the target tooth in the facial image in a first direction is obtained according to the boundary information of the target tooth; where the first direction is associated with the physical width information;
the mapping relationship is determined according to the physical width information and the pixel value.

In a possible implementation, the physical deviation information between the facial midline and the dental midline of the patient is determined according to the mapping relationship, dental midline information of the dental model, and the image deviation information;
or, the facial midline on the dental model is determined according to the mapping relationship, the image deviation information, and dental midline information of the dental model.

In a possible implementation, the facial midline generated is displayed on the dental model.

In a fourth aspect, the present application provides an orthodontic treatment appliance, and the orthodontic treatment appliance is manufactured based on the method for generating the orthodontic treatment plan in any one of the second aspect and the third aspect.

In a fifth aspect, the present application provides a method for manufacturing an orthodontic treatment appliance, the method includes: obtaining an orthodontic treatment plan according to the method for generating the orthodontic treatment plan in any one of the second aspect and the third aspect; manufacturing an orthodontic treatment appliance according to the orthodontic treatment plan.

In a sixth aspect, the present application provides a system for generating a facial midline, the system includes:
an obtaining module, configured to obtain a facial image of a patient;
a processing module, configured to recognize the facial image and determine image deviation information between a facial midline and a dental midline of the patient in the facial image; and
a generating module, configured to generate a facial midline on a dental model of the patient according to the image deviation information and physical parameter information of the dental model.

In a seventh aspect, the present application provides a system for generating an orthodontic treatment plan, the system includes:
an obtaining module, configured to obtain facial midline generated on a dental model, where the facial midline is generated based on the above method for generating the facial midline or generated based on the system for generating the facial midline in the sixth aspect;
a generating module, configured to generate an orthodontic treatment plan for the patient based on the facial midline and the dental model of the patient.

In an eighth aspect, the present application provides a system for generating an orthodontic treatment plan, the system includes:
an obtaining module, configured to obtain a facial image of a patient;
a processing module, configured to recognize the facial image and determine image deviation information between a facial midline and a dental midline of the patient in the facial image; and determine physical deviation information between the facial midline and the dental midline of the patient according to the image deviation information and physical parameter information of a tooth of the patient; and
a generating module, configured to generate an orthodontic treatment plan for the patient based on the physical deviation information and a dental model of the patient.

### Brief Description of Figures

FIG. 1 is a flowchart of a method for generating a facial midline of a three-dimensional dental model according to an embodiment of the present application;
FIG. 2 is a schematic diagram of facial feature positioning points in a method for generating a facial midline in a three-dimensional dental model according to an embodiment of the present application;
FIG. 3 is a schematic diagram of positions of a first facial midline and a first dental midline in a target two-dimensional photograph in a method for generating a facial midline in a three-dimensional dental model according to an embodiment of the present application;
FIG. 4 is a schematic diagram of a three-dimensional dental model in a method for generating a facial midline of a three-dimensional dental model according to an embodiment of the present application;
FIG. 5 is a flowchart of a method for determining a facial midline of a three-dimensional dental model according to an embodiment of the present application;
FIG. 6 is a flowchart of a method for determining a dental orthodontic position according to an embodiment of the present application;
FIG. 7 is a flowchart of a method for manufacturing an orthodontic treatment appliance according to an embodiment of the present application;
FIG. 8 is a schematic structural diagram of a system for determining a facial midline of a three-dimensional dental model according to an embodiment of the present application;
FIG. 9 is a schematic structural diagram of a system for determining a dental orthodontic position according to an embodiment of the present application;
FIG. 10 is a schematic structural diagram of an electronic device according to an embodiment of the present application.

### Detailed Description

The present application is further described below by way of examples, without limiting the scope of the present application to the described examples.

In orthodontics, coordination of a dental midline and a facial midline is one of ideal treatment goals. The dental midline may be obtained in a three-dimensional digital model, and a position of the facial midline in the model is difficult to estimate. One possible way is that the position of the facial midline is mainly obtained by the orthodontic designer observing a front smile photo in combination with personal experience: that is, the position of the facial midline in the dentition is approximately estimated from the front smile photo (such as the facial midline is located at a mesial quarter of a left central incisor), and then is returned to the digital model to obtain by clicking on a corresponding position. This method is not only complicated, but also has certain randomness and error.

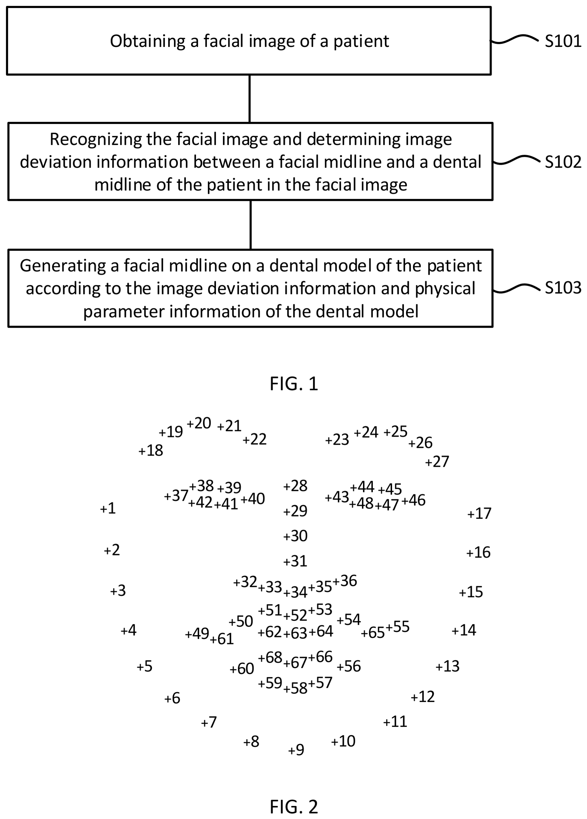

Based on the above problem, embodiments provide a method for generating a facial midline, as shown in FIG. 1, the method includes following steps.

S101: Obtaining a facial image of a patient.

In a possible implementation, a facial image of a patient may be captured during an invisalign procedure of the patient. The facial image is a facial image of a patient in different functional states, including a facial image in a natural smile state, i.e., a front smile. In addition, a three-dimensional digital model of a patient including a crown and a gum may be obtained. The three-dimensional digital model may be obtained by means of a mouth scanning device.

S102: Recognizing the facial image and determining image deviation information between a facial midline and a dental midline of the patient in the facial image.

S103: Generating a facial midline on a dental model of the patient according to the image deviation information and physical parameter information of the dental model.

In a possible implementation, the physical parameter information of the dental model may be used to indicate a variety of parameters, for example, may include size information of a tooth, physical attribute information corresponding to tooth modeling, physical position information corresponding to tooth modeling, etc., and may also represent information such as tooth edge, crown, gum, tooth relative position, tooth jaw relative position, etc., which is not limited herein. Parameter information of the tooth may be obtained in many ways, which may be obtained based on the patient through a mouth scanning device, and may also be obtained from a two-dimensional image, a three-dimensional image, an X-ray image, a CBCT image, or the like, and is not limited herein.

The image deviation information is determined by recognizing the facial image, and the facial midline on the dental model is generated according to the deviation information of the image and the physical parameter information of the dental model of the patient. A relative position relationship between the facial midline and the dental midline in the facial image may be effectively utilized, and combined with the physical parameter information of the dental model, the facial midline on the dental model may be more easily and accurately determined, which is helpful to simplify the design process of orthodontic plan and improve the design efficiency.

A possible implementation is to perform image recognition on the facial image based on a facial region recognition algorithm to obtain a facial region.

For example, the facial image may be one or more target two-dimensional photographs, which is not limited herein.

In a possible implementation, a facial region recognition algorithm may be used to perform image recognition on a target two-dimensional photograph, detect whether a face exist in the target two-dimensional photograph, and mark a facial region. The facial region recognition algorithm may be a conventional machine learning method, such as Haar Cascade, Eigenfaces, Fisherfaces, etc., and may also be a convolutional neural network-based method.

The Haar Cascade algorithm is an object detection algorithm for positioning an object on an image. The algorithm learns from a large number of positive and negative samples based on machine learning. The positive sample includes an object of interest, while the negative sample incudes anything other than an object to be found. A cascade function is trained from a large number of positive and negative images, and then is used to detect an object in other images.

The Eigenfaces algorithm may determine a set of "standardized facial components" through statistical analysis of a large number of facial images. Facial features are assigned constant values because this algorithm does not use digital pictures, but rather a statistical database. Any facial image is a combination of different percentages of these values. This algorithm uses a Principal Component Analysis (PAC) method to process high-dimensional facial data into low-dimensional data. Data analysis and processing are then performed to obtain recognition results, and the PCA method is used to reduce dimensionality of original data, to obtain principal component information thereof to realize the facial recognition method.

The Fisherfaces algorithm is an eigenface-based method that finds a linear combination of features that maximizes variance in data. Eigenface is a name of a set of feature vectors used in the computer vision problem of facial recognition. Eigenfaces is based on PCA (Principal Component Analysis).

In a possible implementation, if a facial region cannot be recognized in the target two-dimensional photograph, a new target two-dimensional photograph needs to be obtained.

In a possible implementation, facial features of the facial image may be recognized to obtain position information of the facial midline of the patient in the facial image.

In a possible implementation, the facial region may be recognized based on a facial feature recognition algorithm, to obtain a facial feature positioning point. The facial feature positioning point may also be referred to as a facial key feature point.

In a possible implementation, after the facial region is recognized, the facial region is recognized by a facial feature recognition algorithm, to obtain a facial feature positioning point. The facial feature recognition algorithm may be a model-based ASM (Active Shape Model) and AAMs (Active Appearance Model), or a cascaded shape regression-based algorithm, and may also be a deep learning-based algorithm.

ASM is a variable model that models shapes (such as facial feature positioning points) in an image. A shape model obtained may be used to analyze a new shape, such as fitting a model to obtain a new shape, and may also be used for generating a shape, such as searching for a shape in a given image.

AAM is an image segmentation algorithm based on an active appearance model. This method may be divided into two parts. The first part is a training part, that is, to construct an active appearance model of a thing, which requires a training set. A function of the training part is to let a program remember a shape feature and an appearance feature of an image to be segmented. The second part is an image segmentation stage. This part is to search features of the thing collected by the training set in the image to be segmented, to find a contour and an appearance of an object image similar to the training set, and then segment the object image from the whole image.

For example, FIG. 2 is a schematic diagram of a facial feature positioning point according to the present embodiment. As shown in FIG. 2, facial feature positioning points 1-17 are facial feature positioning points corresponding to an outer contour of a face, facial feature positioning points 18-27 are facial feature positioning points corresponding to two eyebrows, facial feature positioning points 28-36 are facial feature positioning points corresponding to a bridge of a nose, facial feature positioning points 37-48 are facial feature positioning points corresponding to two eyes, and facial feature positioning points 49-68 are facial feature positioning points corresponding to a lip.

In an optional embodiment, the method may further include determining whether a face of a target object in the target two-dimensional photograph is tilted based on the positions of the facial feature positioning points. If the face of the target object in the target two-dimensional photograph is tilted, rotation correction operation is performed on the facial feature positioning points.

In a possible implementation, in order to prevent the tilt of the face of the target object in the two-dimensional target photograph caused by the change of the shooting angle or the face posture, rotation correction may be performed based on the above facial feature positioning points obtained. Taking the target object as a person as an example, a human facial image coordinate system is established by taking that a center of a line connecting inner corner points of left and right eyes as a coordinate origin. A rotation matrix is obtained by rotating the image to keep the inner corners of the two eyes horizontal, to realize the rotation correction operation on the facial feature positioning points.

In a possible implementation, a rotation correction operation is performed on the facial feature positioning points, so that the facial region of the patient (target object) is no longer tilted, and the face in the facial image (target two-dimensional photograph) is kept upright, which is convenient to mark the position of the dental midline and the position of the facial midline in the target two-dimensional photograph.

In an optional embodiment, facial feature key points may be selected among the facial feature positioning points, and a position of a first facial midline is obtained based on the facial feature key points.

In a possible implementation, the facial feature positioning points may include positioning points corresponding to eyes, eyebrows, a nose bridge, lips and a facial contour. The facial feature key points may include at least three of an eyebrow center point, an eye corner point, a nose tip point, a nose base point, and a philtrum point.

In an optional embodiment, as shown in FIG. 2, the facial feature positioning points 20 and 25 are facial feature key points corresponding to eyebrow center points. The facial feature positioning points 37 and 46 are facial feature key points corresponding to outer eye corner points. The facial feature positioning points 40 and 43 are facial feature key points corresponding to inner eye corner points. The facial feature positioning point 31 is a facial feature key point corresponding to a nose tip point. The facial feature positioning point 34 is a facial feature key point corresponding to a nose base point, and a midpoint of the facial feature positioning points 34 and 52 is taken as a philtrum point.

At least three facial feature key points are selected, and fitting processing is performed on the facial feature positioning points based on a fitting algorithm, to obtain the position of the first facial midline.

In a possible implementation, fitting processing is performed on the facial feature positioning points based on a fitting algorithm, to obtain the position of the first facial midline.

For ease of description, in the following embodiments, the first facial midline is taken as the facial midline of the patient in the facial image determined based on the facial image. Position information of the facial midline of the patient in the facial image may be used to indicate the position of the first facial midline. The position of the first facial midline may be represented based on various data representations, may be coordinate information of the position of the first facial midline, may be based on pixel information corresponding to the pixel position of the facial image, and may also be representation of other position information such as a size value, which is not limit herein.

In a possible implementation, the fitting algorithm includes, but is not limited to, the least square method and the Hough transform method. The basic principle of Hough transform is to transform a curve (including a straight line) in an image space into a parameter space. By detecting an extreme point in the parameter space, a description parameter of the curve is determined, and a regular curve in the image is extracted.

In a possible implementation, the facial feature positioning points are symmetrically distributed on both sides of the first facial midline with the first facial midline as the axis of symmetry.

In a possible implementation, the facial image at least partially includes a partial image of at least one tooth of the patient. The tooth in the facial image is recognized, and position information of the first dental midline is determined.

In a possible implementation, the first dental midline may be a midline between two central incisors of the upper jaw, and may also be a midline between two central incisors of the lower jaw.

In a possible implementation, a tooth in the facial image may be recognized to determine boundary information of a target tooth. Considering facial images of patients in different states, a complete tooth may not be collected. The target tooth may be at least a portion of at least one tooth associated with the facial midline and the dental midline. For example, the target tooth may be one or more teeth near the facial midline or the dental midline. The target tooth may be a portion of one or more teeth near the facial midline or the dental midline. The target tooth may also be a selected tooth capable of displaying a complete width, and the target tooth may also be in other combinations, which is not limit herein.

In a possible implementation, the first dental midline and boundary information of the target tooth may be obtained based on traditional image recognition, such as edge detection, and may also be based on deep learning, such as convolutional neural network.

For example, by taking a midline between two central incisors of the upper jaw or a midline between two central incisors of the lower jaw, and taking the midline as the first dental midline (which may be a first tooth midline), position information of the first dental midline may be obtained, for example, a position of the first tooth midline may be obtained.

In a possible implementation, the boundary information of the target tooth may be used to represent a boundary of a tooth. Considering facial images of patients in different states, a complete tooth may not be collected. The boundary information of the target tooth may also be a boundary of one or more teeth near the central incisor or the facial midline.

In a possible implementation, the boundary information includes pixel information of a dental image.

It should be noted that the boundary information may be represented based on various data representations, may be coordinate information of the position of the boundary of the target tooth, may be based on pixel information corresponding to the pixel position of the facial image, and may also be representation of other position information such as a size value, which is not limited herein.

In a possible implementation, if the target tooth is only one central incisor, a boundary line of the central incisor adjacent to the other central incisor in the same jaw direction is called a mesial boundary line, and the mesial boundary line is taken as the first dental midline. A boundary line of the central incisor away from the other central incisor in the same jaw direction is called a distal boundary line.

By way of example, a first size value of a corresponding central incisor in the target two-dimensional photograph can be calculated according to the mesial boundary line and the distal boundary line.

In a possible implementation, the image deviation information is determined according to the position information of the first facial midline and the position information of the first dental midline. The image deviation information includes: image pixel information of the facial midline and the dental midline.

It should be noted that the image pixel information may be represented based on various data representations, may be image pixel values, the number of image pixels, and may also be representation of other image pixel information determined based on an image size, which is not limited herein.

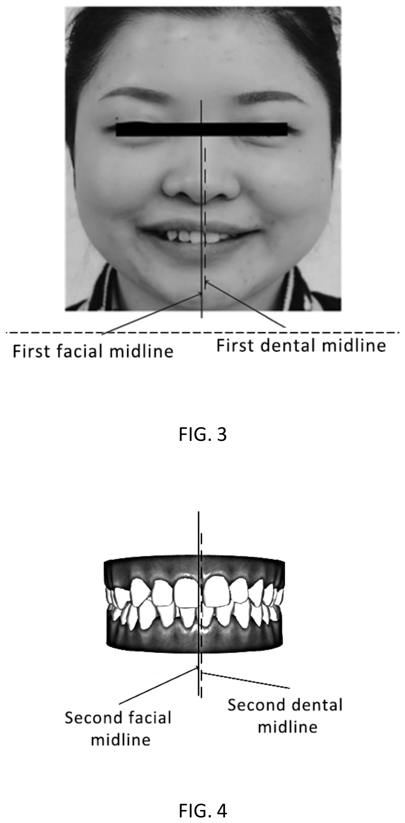

In an optional embodiment, FIG. 3 is a schematic diagram of positions of a first facial midline and a first dental midline in a facial image (for example, a target two-dimensional photograph) according to the present embodiment. FIG. 3 shows positions of the first dental midline and the first facial midline, and the position of the first dental midline is a midline between two central incisors of the upper jaw.

In an optional embodiment, a second dental midline on the dental model may be determined based on the dental model.

For example, by scanning a mouth of a target subject, a three-dimensional dental model of the target object including a crown and a gum is established. For example, in a three-dimensional dental model, a midline between two central incisors in the upper jaw or a midline between two central incisors in the lower jaw is taken as a position of the second dental midline. In a possible implementation, distribution of each tooth of the target object and a size of each tooth may be obtained according to the three-dimensional dental model, and then a second size value of the central incisor is obtained.

In the following, a tooth tangent line determined by the dental model is taken as the second dental midline.

By way of example, the method may include obtaining a three-dimensional dental model of the target object including a crown and a gum. In the three-dimensional dental model, the midline between the two central incisors of the upper jaw or the midline between the two central incisors of the lower jaw is taken as the position of the second dental midline, and a second size value of the corresponding central incisor is obtained.

In a possible implementation, size information of the tooth may be obtained based on a statistical law. For example, depending on the age, sex, general shape and size of a tooth of a target subject, size information of the tooth may be obtained according to a preset size value stored in a tooth database. In this way, the second size value of the central incisor may be determined.

In a possible implementation, physical width information corresponding to the target tooth may be obtained in the dental model. The width information may be determined based on an incisor surface of the target tooth, and may also be determined based on other ways, for example, based on the maximum width of a crown surface of the target tooth identifiable in the facial image. A direction of the width may be different from a direction of the incisor. The width information may be determined by a single tooth, may be determined by a plurality of teeth, and may also be determined based on dental information captured in the facial image, for example, based on a boundary identified in the facial image through a single tooth or a plurality of teeth, and may also be determined in other ways, which is not limited here.

In a possible implementation, physical deviation information between the facial midline and the dental midline of the patient is determined according to the image deviation information and physical size information of a target tooth. The target tooth is at least a portion of at least one tooth associated with the facial midline and the dental midline.

For example, a mapping relationship between an image pixel and a physical space of the dental model is determined according to the image deviation information and the physical size information of the target tooth, and then physical deviation information between the facial midline and the dental midline of the patient is determined based on the mapping relationship.

In an optional embodiment, a mapping relationship is determined according to the boundary information of the target tooth and physical width information of the target tooth. The mapping relationship represents a mapping relationship between an image pixel and a physical space of the dental model. The mapping relationship is used for generating the facial midline on the dental model.

A pixel value occupied by the target tooth in the facial image in a first direction is determined according to determined first direction corresponding to width information of the target tooth; the pixel value occupied by the target tooth in the facial image in the first direction is obtained according to the boundary information of the target tooth. The first direction is related to the physical width information, and the mapping relationship is determined according to the physical width information and the pixel value.

It should be noted that the first direction may include a plurality of directions, and the first direction may be related to the physical width information, and may also not be related to the physical width information. In an optional embodiment, a mapping relationship with width information of a physical model may also be established based on the pixel value occupied by the target tooth in the facial image, to determine the mapping relationship between the image pixel and the physical space of the dental model.

By way of example, a correspondence relationship between the image deviation information and the physical deviation information is determined based on the image deviation information and physical size information of the target tooth, and then the physical deviation information is determined based on the correspondence relationship.

In an optional embodiment, a pixel value of the target tooth in the target two-dimensional photograph is obtained based on an image recognition algorithm, and the pixel value is taken as a first size value. A mapping relationship between a first size value and a second size value is established based on the first size value and the second size value corresponding to the target tooth.

After a mesial boundary and a distal boundary of a central incisor are obtained, a pixel value of the central incisor in the target two-dimensional photograph is calculated based on an image recognition algorithm, and the pixel value is taken as a first size value of the target tooth in the target two-dimensional photograph. At the same time, a second size value of the central incisor may be obtained in the three-dimensional dental model. Based on the first size value corresponding to the central incisor in the target two-dimensional photograph and the second size value corresponding to the central incisor in the three-dimensional dental model, a mapping relationship between the first size value and the second size value is established.

In the method for determining the facial midline in the three-dimensional dental model of the present embodiment, automatic obtaining of the first size value of the target tooth in the target two-dimensional photograph is realized, a mapping relationship between the first size value and the second size value is established based on the first size value and the second size value corresponding to the target tooth, and then the position of the second facial midline on the three-dimensional dental model is automatically determined, improving the accuracy and efficiency of determining the facial midline.

In an optional embodiment, the method further includes: a scale value of a target tooth in a target two-dimensional photograph is obtained based on a preset scale corresponding to the target two-dimensional photograph, and the scale value is taken as the first size value. A mapping relationship between a first size value and a second size value is established based on the first size value and the second size value corresponding to the target tooth.

When the target two-dimensional photograph has a corresponding preset scale, the scale value of the target tooth in the target two-dimensional photo may be obtained according to a numerical value defined by the preset scale, and the scale value is taken as the first size value.

In a possible implementation, the facial midline is generated on the dental model according to physical deviation information between the facial midline and the dental midline of the patient.

In a possible implementation, the physical deviation information between the facial midline and the dental midline of the patient is determined according to the mapping relationship, dental midline information of the dental model and the image deviation information.

In a possible implementation, the facial midline on the dental model is determined according to the mapping relationship, the image deviation information, and dental midline information of the dental model.

It should be noted that the physical deviation information may be based on various data representations, may be coordinate information of the facial midline and the dental midline in the dental model, may be based on corresponding mesh information in the dental model, and may also be representation of other position information such as point cloud data, which is not limited thereto.

In an optional embodiment, after obtaining a first deviation value between the first facial midline and the first dental midline, the first deviation value may be converted into a corresponding second deviation value according to the mapping relationship between the first size value and the second size value. Since the position of the second dental midline has been determined, a relative position of the second facial midline relative to the second dental midline may be obtained according to the second deviation value, and then a position of the second facial midline in the three-dimensional dental model is obtained based on the relative position. A relative position of the second facial midline with respect to the second dental midline is obtained based on the second deviation value. The position of the second facial midline in the three-dimensional dental model is obtained based on the relative position.

In the present embodiment, a position of the second facial midline in the three-dimensional dental model is automatically obtained according to the second deviation value between the second facial midline and the second dental midline of the target object, a mapping relationship between the first size value and the second size value, and the position of the second dental midline in the three-dimensional dental model, improving the accuracy and efficiency of determining the facial midline.

FIG. 4 is a schematic diagram of a three-dimensional dental model according to the present embodiment. FIG. 4 shows a position of a second dental midline and a position of a second facial midline. Through the above method, the position of the second facial midline may be determined on the three-dimensional dental model, and the accuracy and efficiency of determining the facial midline are improved.

In a possible implementation, the facial midline on the dental model may be used for generating or adjust an orthodontic treatment plan for the patient. In a possible implementation, physical deviation information between the facial midline and the dental midline of the patient may be used for generating or adjust an orthodontic treatment plan for the patient. In a possible implementation, the facial midline on the dental model and the physical deviation information between the facial midline and the dental midline of the patient may be used for generating or adjust an orthodontic treatment plan for the patient.

In a possible implementation, the facial midline generated may be displayed on the dental model. In a possible implementation, the physical deviation information generated may be displayed on the dental model. Through the above method, it is helpful to simplify a design process of the orthodontic treatment plan and improve the design efficiency.

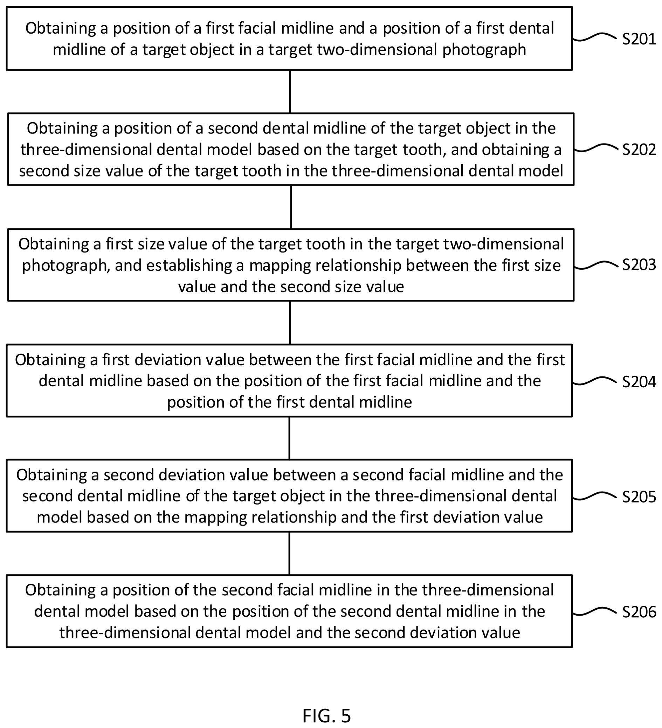

The present embodiment provides a method for determining a facial midline in a three-dimensional dental model. As shown in FIG. 5, the determining method includes following steps.

S201: Obtaining a position of a first facial midline and a position of a first dental midline of a target object in a target two-dimensional photograph.

The target two-dimensional photograph includes a face of the target object with a target tooth exposed.

S202: Obtaining a position of a second dental midline of the target object in the three-dimensional dental model based on the target tooth, and obtaining a second size value of the target tooth in the three-dimensional dental model.

S203: Obtaining a first size value of the target tooth in the target two-dimensional photograph, and establishing a mapping relationship between the first size value and the second size value.

S204: Obtaining a first deviation value between the first facial midline and the first dental midline based on the position of the first facial midline and the position of the first dental midline.

S205: Obtaining a second deviation value between a second facial midline and the second dental midline of the target object in the three-dimensional dental model based on the mapping relationship and the first deviation value.

S206: Obtaining a position of the second facial midline in the three-dimensional dental model based on the position of the second dental midline in the three-dimensional dental model and the second deviation value.

The first dental midline is a dental midline of the target object in the target two-dimensional photograph. The first facial midline is a facial midline of the target object in the target two-dimensional photograph. The second dental midline is a dental midline of the target object in the three-dimensional dental model. The second facial midline is a facial midline of the target object in the three-dimensional dental model.

The second size value is an actual physical size value of the target tooth of the target object, may be obtained through the three-dimensional dental model, and may also be obtained through the statistical law of the tooth.

In the method for determining a facial midline in a three-dimensional dental model of embodiments, the position of the first facial midline and the position of the first dental midline of the target object in the target two-dimensional photograph may be automatically obtained, a mapping relationship between the first size value of the target tooth of the target object in the target two-dimensional photograph and the second size value of the target tooth in the three-dimensional dental model is established according to the first size value and the second size value, then the second deviation value between the second facial midline and the second dental midline of the target object in the three-dimensional dental model is obtained according to the first deviation value between the first facial midline and the first dental midline, and finally the position of the second facial midline in the three-dimensional dental model according to the position of the second dental midline in the three-dimensional dental model and the second deviation value. The position of the facial midline in the three-dimensional dental model is automatically obtained, and the accuracy and efficiency of determining the facial midline are improved.

Positions of the facial midline and the dental midline of the patient are automatically identified based on a front smile photo. At the same time, a pixel size corresponding to a width of a central incisor is automatically detected in an image. Then a physical width size of the central incisor is obtained according to statistical information, to calculate a physical size of each pixel in the front smile photo. Finally, a true deviation between the facial midline and the dental midline in a digital model of the patient is calculated and obtained by using pixel deviation between the facial midline and the dental midline and the physical size of each pixel, to determine position information of the facial midline in the model. The method simulates the process of determining the facial midline by an orthodontic designer, and converts manual identification into automation. Therefore, the facial midline may be determined accurately and efficiently, and the design efficiency is improved.

In an optional embodiment, the method includes: obtaining a target two-dimensional photograph of a target object, obtaining a facial region in the target two-dimensional photograph, obtaining facial feature positioning points in the facial region, obtaining a position of a first facial midline based on the facial feature positioning points, and obtaining a position of a first dental midline based on a position of the target tooth in the target two-dimensional photograph.

The target tooth includes at least one of two central incisors of an upper jaw or at least one of two central incisors of a lower jaw. If the target object is a person, the target two-dimensional photograph may be a front smile photo in which the target tooth is exposed. The central incisor is commonly known as the incisor, and the dental midline may also be called the midline of the dentition. The dental midline is an imaginary line that passes between the two central incisors of the upper jaw or the two central incisors of the lower jaw.

In the method for determining a facial midline in a three-dimensional dental model of embodiments, the position of the first facial midline and the position of the first dental midline of the target object in the target two-dimensional photograph is automatically obtained according to a corresponding algorithm, a mapping relationship between the first size value of the target tooth of the target object in the target two-dimensional photograph and the second size value of the target tooth in the three-dimensional dental model is established according to the first size value and the second size value, then the second deviation value between the second facial midline and the second dental midline of the target object in the three-dimensional dental model is obtained according to the first deviation value between the first facial midline and the first dental midline, and finally the position of the second facial midline in the three-dimensional dental model according to the position of the second dental midline in the three-dimensional dental model and the second deviation value. The position of the facial midline in the three-dimensional dental model is automatically obtained, and the accuracy and efficiency of determining the facial midline are improved.

The present application provides a method for generating an orthodontic treatment plan, the method includes following steps of: obtaining a facial midline generated on a dental model, where the facial midline is generated based on any one possible method for generating a facial midline of the above embodiments; and generating an orthodontic treatment plan for the patient based on the facial midline and the dental model of the patient.

The present application provides a method for generating an orthodontic treatment plan, the method includes following steps of: obtaining a facial image of a patient; recognizing the facial image and determining image deviation information between a facial midline and a dental midline of the patient in the facial image; determining physical deviation information between the facial midline and the dental midline of the patient according to the image deviation information and physical parameter information of a tooth of the patient; and generating an orthodontic treatment plan for the patient based on the physical deviation information and a dental model of the patient.

In a possible implementation, the physical deviation information between the facial midline and the dental midline of the patient is determined according to the image deviation information and the physical size information of the target tooth. The target tooth is at least a portion of at least one tooth associated with the facial midline and the dental midline.

In a possible implementation, the facial midline generated is displayed on the dental model.

The specific embodiment may be any one of the above embodiments, and will not be described here.

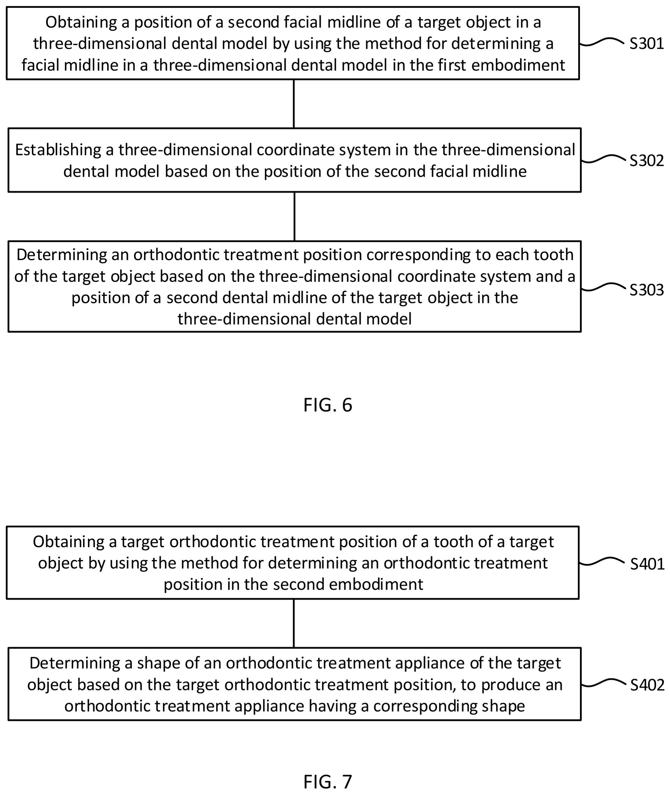

The present embodiment provides a method for determining a dental orthodontic treatment position. As shown in FIG. 6, the method for determining a dental orthodontic treatment position includes following steps.

S301: Obtaining a position of a second facial midline of a target object in a three-dimensional dental model by using the method for determining a facial midline in a three-dimensional dental model in the foregoing embodiment.

S302: Establishing a three-dimensional coordinate system in the three-dimensional dental model based on the position of the second facial midline.

S303: Determining an orthodontic treatment position corresponding to each tooth of the target object based on the three-dimensional coordinate system and a position of a second dental midline of the target object in the three-dimensional dental model.

In the method for determining the dental orthodontic treatment position in the present embodiment, the position of the facial midline of the target object in the three-dimensional dental model is automatically obtained based on the method for determining the facial midline in the three-dimensional dental model in the foregoing embodiment, then the three-dimensional coordinate system is established based on the position of the second facial midline, and the orthodontic treatment position corresponding to each tooth of the target object is determined according to the position of the second dental midline of the target object in the three-dimensional dental model in the three-dimensional coordinate system, such as whether a tooth is moving to the left or to the right relative to the second facial midline.

The method for determining the dental orthodontic treatment position in the embodiment may quickly determine the dental orthodontic treatment position corresponding to each tooth of the target object, to flexibly design and adjust the orthodontic treatment plan.

The present application provides an orthodontic treatment appliance generated using any one method for generating an orthodontic treatment plan described above.

The present application provides a method of manufacturing an orthodontic treatment appliance, obtains an orthodontic treatment plan according to any one method for generating an orthodontic treatment plan described above, and manufactures an orthodontic treatment appliance according to the orthodontic treatment plan.

The present embodiment provides a method for manufacturing an orthodontic treatment plan appliance. As shown in FIG. 7, the method for manufacturing an orthodontic treatment plan appliance includes following steps.

S401: Obtaining a target orthodontic treatment position of a tooth of a target object by using the method for determining an orthodontic treatment position in the foregoing embodiment.

S402: Determining a shape of an orthodontic treatment appliance of the target object based on the target orthodontic treatment position, to produce an orthodontic treatment appliance having a corresponding shape.

In particular, obtained data of a target orthodontic treatment position of a tooth may be transmitted to a device for manufacturing an orthodontic treatment appliance. The device for manufacturing an orthodontic treatment appliance may form a mold (such as a positive mold) for the dental orthodontic treatment appliance according to the data in a general engineering method, so that an orthodontic treatment appliance having a corresponding shape is manufactured from the mold.

Of course, the device for manufacturing an orthodontic treatment appliance may also utilize three-dimensional printing technology to form a positive mold of the dental orthodontic treatment appliance according to the data of the target orthodontic treatment position of the tooth, and further manufacture an orthodontic treatment appliance having a corresponding shape from the positive mold.

In addition, the device for manufacturing an orthodontic treatment appliance may also utilize three-dimensional printing technology to determine the data of the corresponding dental orthodontic treatment appliance according to the data of the target orthodontic treatment position of the tooth, and directly form the dental orthodontic treatment appliance according to the data of the dental orthodontic treatment appliance.

In an optional embodiment, the dental orthodontic treatment appliance is made of a flexible, transparent polymeric material, to form a bracketless invisible orthodontic treatment appliance.

In the method for manufacturing the dental orthodontic treatment appliance according to the present embodiment, the shape of the dental orthodontic treatment appliance of the target object may be quickly and accurately determined according to the target orthodontic treatment position of the tooth of the target object, and then the dental orthodontic treatment appliance having the corresponding shape is manufactured.

In an optional embodiment, the present application provides a system for generating a facial midline, the system includes following modules.

An obtaining module is configured to obtain a facial image of a patient.

A processing module is configured to recognize the facial image and determine image deviation information between a facial midline and a dental midline of the patient in the facial image.

A generating module is configured to generate a facial midline on a dental model of the patient according to the image deviation information and physical parameter information of the dental model.

In an optional embodiment, the present application provides a system for generating an orthodontic treatment plan, the system includes following modules.

An obtaining module is configured to obtain a facial midline generated on a dental model. The facial midline is generated based on the above any one method for generating the facial midline or generated based on the system for generating the facial midline in the sixth aspect.

A generating module is configured to generate an orthodontic treatment plan for the patient based on the facial midline and the dental model of the patient.

In an optional embodiment, the present application provides a system for generating an orthodontic treatment plan, the system including following modules.

An obtaining module is configured to obtain a facial image of a patient.

A processing module is configured to recognize the facial image and determine image deviation information between a facial midline and a dental midline of the patient in the facial image; determine physical deviation information between the facial midline and the dental midline of the patient according to the image deviation information and physical parameter information of a tooth of the patient.

A generating module is configured to generate an orthodontic treatment plan for the patient based on the physical deviation information and a dental model of the patient.

Specific implementations may refer to implementations of the method embodiment in the above embodiment, and will not be described here.

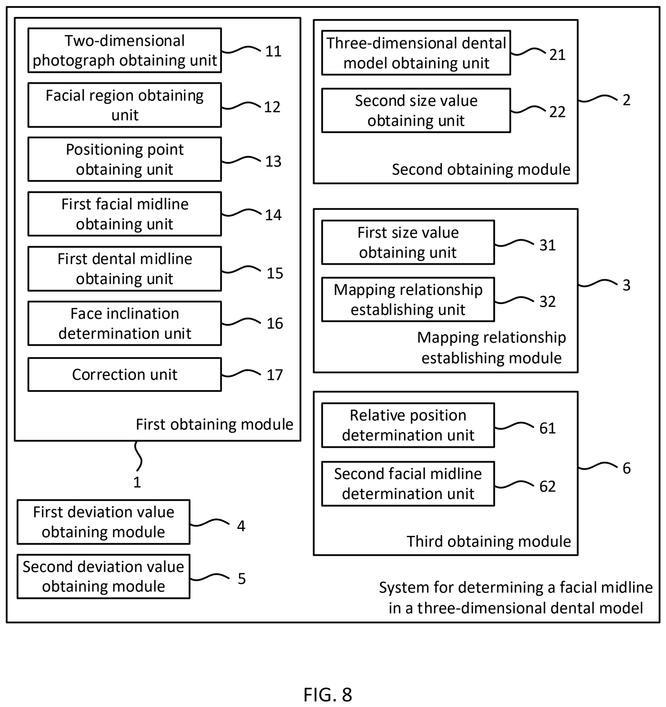

The present embodiment provides a system for determining a facial midline in a three-dimensional dental model, as shown in FIG. 8. The system for determining the facial midline in the three-dimensional dental model includes following modules.

A first obtaining module 1 is configured to obtain a position of a first facial midline and a position of a first dental midline of a target object in a target two-dimensional photograph.

The target two-dimensional photograph includes a face of the target object with a target tooth exposed.

A second obtaining module 2 is configured to obtain a position of a second dental midline of the target object in the three-dimensional dental model based on the target tooth, and obtain a second size value of the target tooth in the three-dimensional dental model.

A mapping relationship establishing module 3 is configured to obtain a first size value of the target tooth in the target two-dimensional photograph, and establish a mapping relationship between the first size value and the second size value.

A first deviation value obtaining module 4 is configured to obtain a first deviation value between the first facial midline and the first dental midline based on the position of the first facial midline and the position of the first dental midline.

A second deviation value obtaining module 5 is configured to obtain a second deviation value between a second facial midline and the second dental midline of the target object in the three-dimensional dental model based on the mapping relationship and the first deviation value.

A third obtaining module 6 is configured to obtain a position of the second facial midline in the three-dimensional dental model based on the position of the second dental midline in the three-dimensional dental model and the second deviation value.

In an optional embodiment, the first obtaining module 1 includes a two-dimensional photograph obtaining unit 11 configured to obtain a target two-dimensional photograph of a target object; a facial region obtaining unit 12 configured to obtain a facial region in the target two-dimensional photograph; a positioning point obtaining unit 13 configured to obtain facial feature positioning points in the facial region; a first facial midline obtaining unit 14 configured to obtain a position of a first facial midline based on the facial feature positioning points; a first dental midline obtaining unit 15 configured to obtain a position of a first dental midline based on a position of a target tooth in the target two-dimensional photograph. The target tooth includes at least one of two central incisors of an upper jaw or at least one of two central incisors of a lower jaw.

In an optional embodiment, the first obtaining module 1 further includes a face inclination determination unit 16 configured to determine whether the face of the target object in the target two-dimensional photograph is inclined or not based on positions of the facial feature positioning points; a correction unit 17 configured to perform a rotation correction operation on the facial feature positioning points when the face inclination determination unit 16 determines that the face of the target object in the target two-dimensional photograph is inclined, and operate a first facial midline obtaining unit 14; operate the first facial midline obtaining unit 14 when the face inclination determination unit 16 determines that the face of the target object in the target two-dimensional photograph is not inclined.

In an optional embodiment, the first facial midline obtaining unit 14 is configured to select facial feature key points from the facial feature positioning points, and obtain the position of the first facial midline based on the facial feature key points. The facial feature positioning points include positioning points corresponding to eyes, eyebrows, a nose bridge, lips and a facial contour. The facial feature key points include at least three of an eyebrow center point, an eye corner point, a nose tip point, a nose base point and a philtrum point.

In an optional embodiment, the facial region obtaining unit 12 is configured to perform image recognition on the target two-dimensional photograph based on a facial region recognition algorithm, to obtain the facial region.

In an optional embodiment, the positioning point obtaining unit 13 is configured to recognize the facial region based on a facial feature recognition algorithm, to obtain the facial feature positioning points.

In an optional embodiment, the first facial midline obtaining unit 14 is configured to perform fitting processing on the facial feature positioning points based on a fitting algorithm, to obtain the position of the first facial midline.

In an optional embodiment, the first dental midline obtaining unit 15 is configured to obtain a midline between two central incisors of the upper jaw or a midline between two central incisors of the lower jaw, and take the midline as the first dental midline to obtain the position of the first dental midline.

In an optional embodiment, the second obtaining module 2 includes a three-dimensional dental model obtaining unit 21 configured to obtain a three-dimensional dental model of the target object including a crown and a gum; a second size value obtaining unit 22 configured to take the midline between the two central incisors of the upper jaw or the midline between the two central incisors of the lower jaw in the three-dimensional dental model as the position of the second dental midline, and obtain a second size value of a corresponding central incisor.

In an optional embodiment, the second obtaining module 2 includes a three-dimensional dental model obtaining unit 21 configured to obtain a three-dimensional dental model of the target object including a crown and a gum; a second size value obtaining unit 22 configured to take the midline between the two central incisors of the upper jaw or the midline between the two central incisors of the lower jaw in the three-dimensional dental model as the position of the second dental midline, and obtain a second size value of a corresponding central incisor based on a preset size value.

In an optional embodiment, the mapping relationship establishing module 3 includes a first size value obtaining unit 31 configured to obtain the first size value of the target tooth in the target two-dimensional photo based on an image recognition algorithm; a mapping relationship establishing unit 32 configured to establish a mapping relationship between the first size value and the second size value based on the first size value and the second size value corresponding to the target tooth.

In an optional embodiment, the mapping relationship establishing module 3 includes a first size value obtaining unit 31 configured to obtain the first size value of the target tooth in the target two-dimensional photograph based on a preset scale corresponding to the target two-dimensional photograph; a mapping relationship establishing unit 32 configured to establish a mapping relationship between the first size value and the second size value based on the first size value and the second size value corresponding to the target tooth.

In an optional embodiment, the third obtaining module 6 includes a relative position determination unit 61 configured to obtain a relative position of the second facial midline with respect to the second dental midline based on the second deviation value; a second facial midline determination unit 62 configured to obtain the position of the second facial midline in the three-dimensional dental model based on the relative position.

The working principle of the system for determining the facial midline in the three-dimensional dental model in the present embodiment is the same as the working principle of the method for determining the facial midline in the three-dimensional dental model according to the foregoing embodiments, and will not be repeated here.

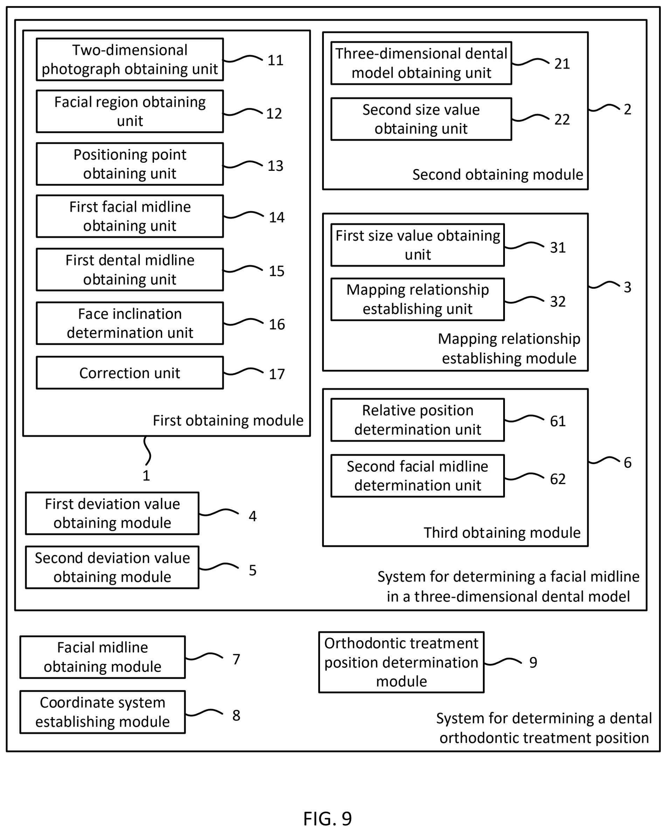

The present embodiment provides a system for determining a dental orthodontic treatment position. As shown in FIG. 9, the system for determining the dental orthodontic treatment position includes following modules.

A facial midline obtaining module 7 is configured to obtain a position of a facial midline of a target object in a three-dimensional dental model using the system for determining the facial midline in the three-dimensional dental model in the previous embodiment.

A coordinate system establishing module 8 is configured to establish a three-dimensional coordinate system in the three-dimensional dental model based on the position of the facial midline.

An orthodontic treatment position determination module 9 is configured to determine an orthodontic treatment position corresponding to each tooth of the target object based on the three-dimensional coordinate system and a position of a dental midline of the target object in the three-dimensional dental model.

The working principle of the system for determining the dental orthodontic treatment position according to the present embodiment is the same as the working principle of the method for determining the dental orthodontic treatment position according to the foregoing embodiments, and will not be repeated here.

In the system for determining the dental orthodontic treatment position of the present embodiment, a position of a facial midline of a target object in a three-dimensional dental model is automatically obtained based on the system for determining the facial midline in the three-dimensional dental model in the previous embodiment, an orthodontic treatment position corresponding to each tooth of the target object may be quickly determined, to flexibly design and adjust the orthodontic treatment plan.

The present embodiment provides a system for manufacturing an orthodontic treatment appliance. The system for manufacturing an orthodontic treatment appliance includes following modules.

An orthodontic treatment position obtaining module is configured to obtain a target orthodontic treatment position of a tooth of a target object using the system for determining a dental orthodontic treatment position in aforementioned embodiments.

An orthodontic treatment appliance manufacturing module is configured to determine a shape of an orthodontic treatment appliance of the target object based on the target orthodontic treatment position, to produce an orthodontic treatment appliance having a corresponding shape.

The working principle of the system for manufacturing the dental orthodontic treatment appliance of the present embodiment is the same as the working principle of the method for manufacturing the dental orthodontic treatment appliance of the above embodiments, and is not repeated herein.

In the system for manufacturing the dental orthodontic treatment appliance of the present embodiment, the shape of the dental orthodontic treatment appliance of the target object may be quickly and accurately determined based on the target orthodontic treatment position of the tooth of the target object determined by the system for determining the dental orthodontic treatment position of the above embodiments, and then the dental orthodontic treatment appliance having the corresponding shape is manufactured.

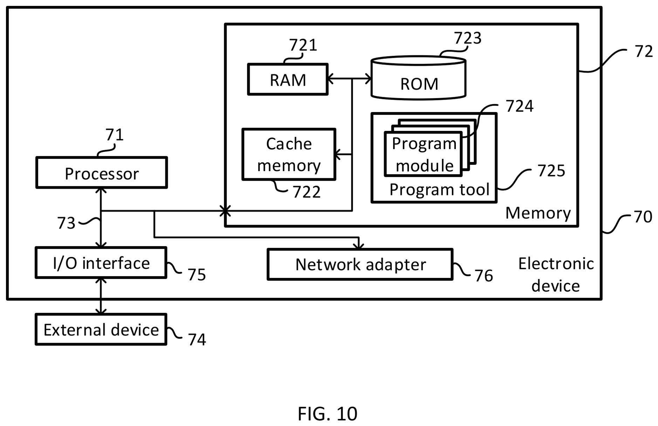

The present embodiment provides an electronic device, and FIG. 10 is a schematic structural diagram of the electronic device according to the present embodiment. The electronic device includes a memory, a processor and a computer program stored on the memory and executable on the processor. The methods in the above embodiments are implemented when the processor executes the computer program. The electronic device shown in FIG. 10 is only an example, and should not limit functions and scope of use of embodiments of the present application. As shown in FIG. 10, the electronic device may be embodied in the form of a general-purpose computing device, for example, may be a server device. Components of the electronic device may include, but are not limited to, the aforementioned at least one processor, the aforementioned at least one memory, a bus connecting different system components (including the memory 72 and the processor).

The bus 73 includes a data bus, an address bus, and a control bus.

The memory 72 may include a volatile memory, such as a random-access memory (RAM) 721 and/or a cache memory 722, and may further include a read-only memory (ROM) 723.

The memory 72 may also include a program tool 725 (or utility) having a set (at least one) of program modules 724. The program modules 724 include, but are not limited to, an operating system, one or more applications, other program modules and program data. Each of these examples, or some combination thereof, may include an implementation of a network environment.

By running the computer program stored in the memory 72, the processor 71 executes various functional applications and data processing, such as the method for determining the facial midline in the three-dimensional dental model in the foregoing embodiments, or the method for determining the dental orthodontic treatment position in the foregoing embodiments, or the method for manufacturing the dental orthodontic treatment appliance in the foregoing embodiments.

The electronic device 70 may also communicate with one or more external devices 74. Such communication may be performed through an input/output (I/O) interface 75. Also, the model generation electronic device 70 may communicate with one or more networks (e.g., a local area network (LAN), a wide area network (WAN) and/or a public network, such as the Internet). As shown in FIG. 10, a network adapter 76 communicates with other modules of the electronic device 70 through the bus 73. It should be understood that although not shown, other hardware and/or software modules may be used in conjunction with the electronic device 70, include, but are not limited to, microcode, device drivers, redundant processors, external disk drive arrays, RAID (disk array) systems, tape drivers and data backup storage systems.

Input and/or output devices may include a scanning device, a camera interface, an input device (e.g., a mouse, a keyboard, etc.), a display device (e.g., a monitor), a printer, and/or one or more other input devices. The input/output interface may receive executable instructions and/or data, which may be stored in a data storage device (e.g., a memory), for example, may be used to receive the facial midline generated and store the facial midline generated.

In some embodiments, the scanning device may be configured to scan one or more physical dental casts of a dentition of a patient. In one or more embodiments, the scanning device may be configured to directly scan the dentition and/or an orthodontic treatment appliance of the patient. The scanning device may be configured to input data into a computing device.

In some embodiments, the camera interface may receive input from an imaging device (e.g., a 2D or 3D imaging device), such as a digital camera, a printed photograph scanner, and/or other suitable imaging device. For example, the input from the imaging device may be stored in the memory.

The processor may execute instructions to provide a display of a facial midline, a dental orthodontic treatment position, a treatment plan, etc. on a display. For example, the computing device may be configured to allow a treatment professional or other user to input treatment goals. The input received may be sent as data to the processor and/or may be stored in the memory.

Such connectivity may allow for input and/or output of data and/or instructions as well as other types of information. Some embodiments may be distributed among various computing devices within one or more networks, for collecting, calculating, and/or analyzing any of the methods mentioned herein.

It should be noted that although several units/modules or sub-units/modules of the electronic device are mentioned in the above detailed description, such division is merely exemplary and not mandatory. Indeed, according to embodiments of the present application, features and functions of two or more of the units/modules described above may be embodied in one unit/module. Conversely, features and functions of one unit/module described above may be further divided to be embodied by a plurality of units/modules.

The present embodiment provides a computer-readable storage medium storing a computer program thereon. The computer program, when executed by a processor, implements the methods in the above-described embodiments.

In a possible embodiment, the present application may also be embodied in the form of a program product including program code. When the program product is run on a terminal device, the program code is for causing the terminal device to perform steps in embodiments described above.

The program code for performing the present application may be written in any combination of one or more programming languages. The program code may be executed completely on a user device, partly on the user device, as a stand-alone software package, partially on the user device and partially on a remote device, or completely on the remote device.

Although embodiments of the present application have been described above, those skilled in the art should understand that this is only an example and the scope of protection of the present application is limited by the appended claims. Those skilled in the art may make various changes or modifications to these embodiments without departing from the principles and essence of the present application, but these changes and modifications fall within the scope of protection of the present application.

## Claims

1. A method for generating a facial midline, the method comprising:
obtaining a facial image of a patient;
recognizing the facial image and determining image deviation information between a facial midline and a dental midline of the patient in the facial image; and
generating a facial midline on a dental model of the patient according to the image deviation information and physical parameter information of the dental model.

2. The method according to claim 1, wherein the physical parameter information comprises physical size information, and the generating the facial midline on the dental model of the patient according to the image deviation information and the physical parameter information of the dental model, comprises:
determining physical deviation information between the facial midline and the dental midline of the patient according to the image deviation information and physical size information of a target tooth;
generating the facial midline on the dental model according to physical deviation information between the facial midline and the dental midline of the patient.

3. The method according to claim 1 or 2, wherein the target tooth is at least a portion of at least one tooth associated with the facial midline and the dental midline.

4. The method according to any one of claims 1 to 3, wherein the facial image at least partially comprises a partial image of at least one tooth of the patient; the determining the image deviation information between the facial midline and the dental midline of the patient in the facial image, comprises:
recognizing facial features of the facial image and obtaining position information of the facial midline of the patient in the facial image;
recognizing the tooth in the facial image and determining position information of the dental midline;
determining the image deviation information according to the position information of the facial midline and the position information of the dental midline, wherein the image deviation information comprises: image pixel information of the facial midline and the dental midline.

5. The method according to any one of claims 2 to 4, the method further comprising:
recognizing a tooth in the facial image and determining boundary information of the target tooth; wherein the boundary information comprises: pixel information of a dental image;
determining a mapping relationship according to the boundary information of the target tooth and physical width information of the target tooth, wherein the mapping relationship represents a mapping relationship between an image pixel and a physical space of the dental model; the mapping relationship is used for generating the facial midline on the dental model.

6. The method according to claim 5, wherein the determining the mapping relationship according to the boundary information of the target tooth and the physical width information of the target tooth, comprises:
obtaining the physical width information corresponding to the target tooth in the dental model;
obtaining a pixel value occupied by the target tooth in the facial image in a first direction according to the boundary information of the target tooth; wherein the first direction is associated with the physical width information;
determining the mapping relationship according to the physical width information and the pixel value.

7. The method according to any one of claims 2 to 6, the method further comprising:
determining the physical deviation information between the facial midline and the dental midline of the patient according to the mapping relationship, dental midline information of the dental model, and the image deviation information;
or, determining the facial midline on the dental model according to the mapping relationship, the image deviation information, and dental midline information of the dental model.

8. The method according to any one of claims 1 to 7, wherein
the facial midline on the dental model is used for generating or adjusting an orthodontic treatment plan for the patient;
and/or, the physical deviation information between the facial midline and the dental midline of the patient is used for generating or adjusting an orthodontic treatment plan for the patient.

9. The method according to any one of claims 1 to 7, the method further comprising:
displaying the facial midline generated on the dental model.

10. A method for generating an orthodontic treatment plan, the method comprising:
obtaining a facial midline generated on a dental model, wherein the facial midline is generated based on the method for generating the facial midline according to any one of claims 1 to 8; and
generating an orthodontic treatment plan for the patient according to the facial midline and the dental model of the patient.

11. A method for generating an orthodontic treatment plan, the method comprising:
obtaining a facial image of a patient;
recognizing the facial image and determining image deviation information between a facial midline and a dental midline of the patient in the facial image;
determining physical deviation information between the facial midline and the dental midline of the patient according to the image deviation information and physical parameter information of a tooth of the patient; and
generating an orthodontic treatment plan for the patient based on the physical deviation information and a dental model of the patient.

12. The method according to claim 11, wherein the determining the physical deviation information between the facial midline and the dental midline of the patient according to the image deviation information and the physical parameter information of the tooth of the patient, comprises:
determining the physical deviation information between the facial midline and the dental midline of the patient according to the image deviation information and physical size information of a target tooth, wherein the target tooth is at least a portion of at least one tooth associated with the facial midline and the dental midline.

13. The method according to claim 11 or 12, wherein the facial image at least partially comprises a partial image of at least one tooth of the patient; the determining the image deviation information between the facial midline and the dental midline of the patient in the facial image comprises:
recognizing facial features of the facial image and obtaining position information of the facial midline of the patient in the facial image;
recognizing the tooth in the facial image and determining position information of the dental midline;
determining the image deviation information according to the position information of the facial midline and the position information of the dental midline, wherein the image deviation information comprises: image pixel information of the facial midline and the dental midline.

14. The method according to any one of claims 11 to 13, the method further comprising:
recognizing a tooth in the facial image and determining boundary information of a target tooth; wherein the boundary information comprises: pixel information of a dental image;
determining a mapping relationship according to the boundary information of the target tooth and physical width information of the target tooth, wherein the mapping relationship represents a mapping relationship between an image pixel and a physical space of the dental model; the mapping relationship is used for generating the facial midline on the dental model.

15. The method according to claim 14, wherein the determining the mapping relationship according to the boundary information of the target tooth and the physical width information of the target tooth, comprises:
obtaining the physical width information corresponding to the target tooth in the dental model;
obtaining a pixel value occupied by the target tooth in the facial image in a first direction according to the boundary information of the target tooth; wherein the first direction is associated with the physical width information;
determining the mapping relationship according to the physical width information and the pixel value.

16. The method according to any one of claims 11 to 15, the method further comprising:
determining the physical deviation information between the facial midline and the dental midline of the patient according to the mapping relationship, dental midline information of the dental model, and the image deviation information;
or, determining the facial midline on the dental model according to the mapping relationship, the image deviation information, and dental midline information of the dental model.

17. The method according to any one of claims 11 to 16, the method further comprising:
displaying the facial midline generated on the dental model.

18. An orthodontic treatment appliance, wherein the orthodontic treatment appliance is manufactured based on the method for generating the orthodontic treatment plan according to claim 10 or any one of claims 11 to 17.

19. A method for manufacturing an orthodontic treatment appliance, the method comprising:
obtaining an orthodontic treatment plan according to the method for generating the orthodontic treatment plan according to claim 10 or any one of claims 11 to 17;
manufacturing an orthodontic treatment appliance according to the orthodontic treatment plan.

20. An electronic device comprising a memory, a processor, and a computer program stored on the memory for execution on the processor, wherein the processor, when executing the computer program, implements the method according to any one of claims 1 to 10, or the method according to any one of claims 11 to 17, or the method according to claim 19.

21. A computer-readable storage medium storing a computer program thereon, wherein the computer program, when executed by a processor, implements the method according to any one of claims 1 to 10, or the method according to any one of claims 11 to 17, or the method according to claim 19.

22. A system for generating a facial midline, the system comprising:
an obtaining module, configured to obtain a facial image of a patient;
a processing module, configured to recognize the facial image and determine image deviation information between a facial midline and a dental midline of the patient in the facial image; and
a generating module, configured to generate a facial midline on a dental model of the patient according to the image deviation information and physical parameter information of the dental model.

23. A system for generating an orthodontic treatment plan, the system comprising:
an obtaining module, configured to obtain facial midline generated on a dental model, wherein the facial midline is generated based on the method for generating the facial midline according to any one of claims 1 to 8 or generated based on the system for generating the facial midline according to claim 22;
a generating module, configured to generate an orthodontic treatment plan for the patient based on the facial midline and the dental model of the patient.

24. A system for generating an orthodontic treatment plan, the system comprising:
an obtaining module, configured to obtain a facial image of a patient;
a processing module, configured to recognize the facial image and determine image deviation information between a facial midline and a dental midline of the patient in the facial image; and determine physical deviation information between the facial midline and the dental midline of the patient according to the image deviation information and physical parameter information of a tooth of the patient; and
a generating module, configured to generate an orthodontic treatment plan for the patient based on the physical deviation information and a dental model of the patient.
